# EUROPEAN PATENT APPLICATION

(11) **EP 4 282 431 A1**
(43) Date of publication of application: **29.11.2023**
(21) Application number: 22742662.4
(22) Date of filing: 20.01.2022
(51) Int. Cl.: A61K 45/00

(54) **NON-FLAMMABLE LIQUID COMPOSITION AND USE OF SAME**

(30) Priority: 25.01.2021 JP 2021009596
(71) Applicant: Tosoh Corporation, Yamaguchi 746-8501 (JP)
(72) Inventor: OKA Yuji, Shunan-shi Yamaguchi 746-8501 (JP); KAWABE Kosuke, Shunan-shi Yamaguchi 746-8501 (JP); KANBARA Takeshi, Shunan-shi Yamaguchi 746-8501 (JP)
(74) Representative: TBK
(86) International application number: PCT/JP2022/002064
(87) International publication number: WO 2022/158543

(57) **Abstract**

To provide an industrially and environmentally more preferable nonflammable liquid composition having a fluorine compound reduced, as compared to previously known nonflammable liquid compositions.

A liquid composition is used, which comprises a flammable liquid and a fluoroiodoalkane represented by the following formula (1): 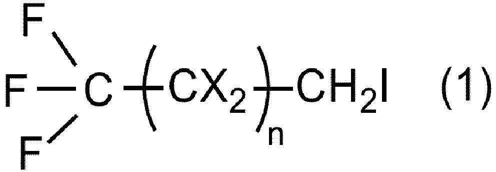 (in the formula, X each independently represents H or F, and n represents an integer of from 0 to 5), and which is characterized in that the total amount of the above-mentioned flammable liquid and the above-mentioned fluoroiodoalkane is from 95 to 100 mass% of the entire amount of the above-mentioned liquid composition.

## Description

### TECHNICAL FIELD

The present invention relates to a nonflammable liquid composition which can be used for a wide range of applications, such as a disinfectant, a cleaning solvent and a dilution application solvent.

### BACKGROUND ART

An organic solvent commonly used in industrial and household applications has a low flash point and is thus likely to cause a fire hazard during transportation, use and storage.

In order to safely transport, use and store such an organic solvent, a nonflammable organic solvent composition having no flash point has been proposed (Patent Document 1).

### PRIOR ART DOCUMENT

### PATENT DOCUMENT

Patent Document 1: JP-A-2020-29533

### DISCLOSURE OF INVENTION

### TECHNICAL PROBLEM

In Patent Document 1, a composition having a fluorinated compound added to achieve nonflammability of the organic solvent is proposed, but nonflammability requires the addition of a large amount of the fluorinated compound. Since the fluorinated compound has high values of the ozone depletion potential (ODP) and global warming potential (GWP), from the viewpoint of environmental preservation, a method that enables nonflammability with a smaller amount added has been desired.

### SOLUTION TO PROBLEM

The present inventors have conducted a diligent study to solve the aforementioned problem and, as a result, have found that by adding a fluoroiodoalkane represented by the formula as described later, it is possible to make a flammable liquid to be nonflammable under a condition of a smaller amount of the addition as compared to the conventional known technology, and thus, have arrived at the completion of the present invention.

That is, the present invention resides in the following [1] to [16].

[1] A liquid composition comprising a flammable liquid and a fluoroiodoalkane represented by the following formula (1): 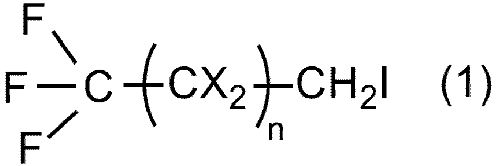 (in the formula, X each independently represents H or F, and n represents an integer of from 0 to 5), characterized in that the total amount of the above-mentioned flammable liquid and the above-mentioned fluoroiodoalkane is from 95 to 100 mass% of the entire amount of the above-mentioned liquid composition.
[2] The liquid composition according to [1], wherein the fluoroiodoalkane represented by the above-mentioned formula (1) is 1,1,1-trifluoro-2-iodoethane, 1,1,1 -trifluoro-3-iodopropane, or 1,1,1-trifluoro-4-iodobutane.
[3] The liquid composition according to [1] or [2], wherein the above-mentioned flammable liquid is a flammable liquid made of at least one type selected from the group consisting of an alcohol compound which may be halogenated, a hydrocarbon compound which may be halogenated, an ether compound which may be halogenated, an epoxy compound which may be halogenated, an amine compound which may be halogenated, and an aromatic compound which may be halogenated.
[4] The liquid composition according to [1] or [2], wherein the above-mentioned flammable liquid is a flammable liquid made of at least one type selected from the group consisting of 2,2,2-trifluoroethanol, ethanol, a 70 vol% ethanol aqueous solution, 1-bromopropane, 1-bromo-2-methylpropane, 2-chloroethyl methyl ether, dichloromethyl methyl ether, toluene, 2-bromo-2-butene, 2-bromopentane, 2,3-dibromopropene, 1,2-dichloroethylene, decane, cyclohexane, 1,2-butylene oxide, 1,2-epoxypentane, 1,2-epoxycyclopentane, trifluoromethylbenzene, 1,2-epoxy-4-vinylcyclohexane, 2,2,6,6-tetramethylpiperidine, and 1,2,2,6,6-pentamethyl-4-piperidyl methacrylate.
[5] The liquid composition according to any one of [1] to [4], wherein in the total amount of the above-mentioned flammable liquid and fluoroiodoalkane, the proportion of the above-mentioned fluoroiodoalkane is from 0.1 to 50 mass%.
[6] The liquid composition according to any one of [1] to [5], wherein the above-mentioned flammable liquid is a flammable liquid containing at least 1-bromo-2-methylpropane and trifluoromethylbenzene.
[7] The liquid composition according to any one of [1] to [6], wherein the above-mentioned flammable liquid is a flammable liquid containing at least 1-bromo-2-methylpropane, trifluoromethylbenzene and 1,2-epoxycyclopentane.
[8] The liquid composition according to any one of [1] to [6], wherein the above-mentioned flammable liquid is a flammable liquid containing at least 1-bromo-2-methylpropane, trifluoromethylbenzene, 1,2-epoxycyclopentane and 1,2,2,6,6-pentamethyl-4-piperidyl methacrylate.
[9] The liquid composition according to any one of [5] to [8], wherein the content of the above-mentioned 1-bromo-2-methylpropane is from 70 to 95 wt% of the entire amount of the above-mentioned liquid composition.
[10] The liquid composition according to any one of [5] to [8], wherein the content of the above-mentioned trifluoromethylbenzene is from 1 to 10 wt% of the entire amount of the above-mentioned liquid composition.
[11] The liquid composition according to [7] or [8], wherein the content of the above-mentioned 1,2-epoxycyclopentane is from 0.5 to 5 wt% of the entire amount of the above-mentioned liquid composition.
[12] The liquid composition according to [8], wherein the content of the above-mentioned 1,2,2,6,6-pentamethyl-4-piperidyl methacrylate is from 0.1 to 2 wt% of the entire amount of the above-mentioned liquid composition.
[13] The liquid composition according to any one of [1] to [12], wherein the content of the above-mentioned fluoroiodoalkane is from 2 to 15 wt% of the entire amount of the above-mentioned liquid composition.
[14] A method of making a flammable liquid nonflammable, characterized by mixing a fluoroiodoalkane represented by the following formula (1): 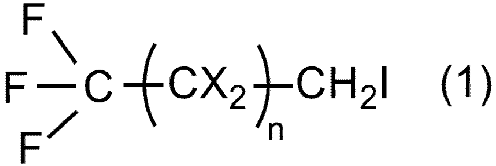 (in the formula, X each independently represents H or F, and n represents an integer of from 0 to 5) to the flammable liquid to change the above-mentioned flammable liquid to a nonflammable liquid.
[15] A washing method characterized by bringing the liquid composition as defined in any one of [1] to [13] into contact with an article to be washed to which an oily component is attached, to wash off the oily component from the above-mentioned article to be washed.
[16] A disinfectant consisting of the liquid composition as defined in any one of [1] to [13].

### ADVANTAGEOUS EFFECTS OF INVENTION

As compared to previously known nonflammable liquid compositions, the present invention can provide a nonflammable liquid composition that is industrially and environmentally preferable because of its superior stability and solubility and the reduced amount of the nonflammable agent added.

Further, the present invention can solve an environmental protection problem by keeping ozone depletion potential (ODP) and global warming potential (GWP) lower as compared to previously known nonflammable liquid compositions.

### DESCRIPTION OF EMBODIMENTS

In the following, the present invention will be specifically described.

The present invention relates to a liquid composition comprising a flammable liquid and a floroiodoalkane represented by the following formula (1): 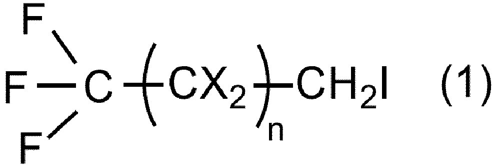 (in the formula, X each independently represents H or F, and n represents an integer of from 0 to 5), characterized in that the total amount of the above-mentioned flammable liquid and the above-mentioned fluoroiodoalkane is from 95 to 100 mass% of the entire amount of the above-mentioned liquid composition.

As the fluoroiodoalkane represented by the above-mentioned formula (1), although not particularly limited thereto, for example, 1,1,1-trifluoro-2-iodoethane, 1,1,1-trifluoro-3-iodopropane, 1,1,1,2-tetrafluoro-3-iodopropane, 1,1,1,2,2-pentafluoro-3-iodopropane, 1,1,1-trifluoro-4-iodobutane, 1,1,1,2,-tetrafluoro -4-iodobutane, 1,1,1,2,2-pentafluoro-4-iodobutane, 1H, 1H-tridecafluoro-1-iodoheptane, etc. may be mentioned. Among these, from such a viewpoint that a high nonflammable effect will be obtained, 1,1,1-trifluoro-2-iodoethane, 1,1,1-trifluoro-3 -iodopropane, 1,1,1-trifluoro-4-iodobutane, or 1,1,1,2,2-pentafluoro-3-iodopropane is preferred, and 1,1,1-trifluoro-2-iodoethane, 1,1,1-trifluoro-3-iodopropane, or 1,1,1-trifluoro-4-iodobutane is more preferred, and 1,1,1-trifluoro-3-iodopropane is further preferred.

Further, with respect to the above-mentioned fluoroiodoalkane, the above-mentioned specific fluoroiodoalkane may be used alone, or two or more specific fluoroiodoalkanes may be combined to form a composition.

In the present invention, the flammable liquid means an article listed in the "Articles" column of Class 4 of Appended Table 1 of the Fire Defense Law of Japan that has the characteristics of a flammable liquid (a liquid having a flash point that falls under Class 4 hazardous materials), and consists of special flammable, alcohol, primary petroleum, secondary petroleum, tertiary petroleum, quaternary petroleum, and animal and vegetable oils.

The above-mentioned special flammable refers to one having a flash point of at most -20°C and a boiling point of at most 40°C at 1 atm, or one having a flash point of less than 100°C and an ignition point of at most 100°C. As the specific example, diethyl ether, carbon disulfide, collodione, acetaldehyde, propylene oxide or pentane may be mentioned.

The above-mentioned alcohol refers to a C₁₋₃ saturated monovalent alcohol, and as the specific example, methyl alcohol, ethyl alcohol, or isopropyl alcohol may be mentioned.

The above-mentioned primary petroleum refers to one having a flash point of less than 21°C at 1 atm (but not including special flammable and the above-mentioned alcohol), and as the specific example, acetone, gasoline, petroleum benzine, ligroin, dioxane, benzene, toluene, petroleum ether, hexane, tetrahydrofuran, isopropyl ether, acrylonitrile, ethylamine, ethyl acetate, or methyl ethyl ketone may be mentioned.

The above-mentioned secondary petroleum refers to one having a flash point of at least 21°C and less than 70°C at 1 atm (but not including the above-mentioned alcohol), and as the specific example, kerosene, diesel oil, chlorobenzene, ethylbenzene, styrene, xylene, ethyl cellosolve, formic acid, acetic acid, turpentine oil, or pine oil may be mentioned.

The above-mentioned tertiary petroleum refers to one having a flash point of at least 70°C and less than 200°C at 1 atm (but not including the above-mentioned alcohol), and as the specific example, heavy oil, creosote oil, glycerin, aniline, nitrobenzene, ethanolamine, ethylene glycol, or cresol may be mentioned.

The above-mentioned quaternary petroleum refers to one having a flash point of at least 200°C at 1 atm, and as the specific example, gear oil, cylinder oil, lubricating oil, turbine oil, machine oil, or motor oil may be mentioned.

The above-mentioned animal and vegetable oils refer to oils derived from animals and plants, and as the specific example, coconut oil, olive oil, castor oil, peanut oil, rape seed oil, sesame oil, or cottonseed oil may be mentioned.

In the present invention, as such a flammable liquid, although not particularly limited thereto, for example, an alcohol compound which may be halogenated, a hydrocarbon compound which may be halogenated, an ether compound which may be halogenated, a ketone compound which may be halogenated, an ester compound which may be halogenated, an epoxy compound which may be halogenated, an amine compound which may be halogenated, or an aromatic compound which may be halogenated, may be mentioned.

Further, with respect to such a flammable liquid, from such a viewpoint that the merit of the nonflammability is large, it is preferable that it is the above-mentioned alcohol, primary petroleum, secondary petroleum, or tertiary petroleum; it is more preferable that it is the above-mentioned primary petroleum, secondary petroleum, or tertiary petroleum; and it is further preferable that it is a flammable liquid made of at least one type selected from the group consisting of an alcohol compound which may be halogenated, a hydrocarbon compound which may be halogenated, an ether compound which may be halogenated, an epoxy compound which may be halogenated, an amine compound which may be halogenated, and an aromatic compound which may be halogenated, corresponding thereto (a mixture of two or more flammable liquids may be referred to also as a flammable liquid composition).

As the above-mentioned alcohol compound which may be halogenated, although not particularly limited, for example, methanol (an alcohol), ethanol (an alcohol), 70 vol% ethanol aqueous solution (an alcohol), 1-propanol (an alcohol), 2 -propanol (an alcohol), 2,2,2-trifluoroethanol (secondary petroleum), 1-butanol (secondary petroleum), or 2-butanol (secondary petroleum), may be mentioned.

As the above-mentioned hydrocarbon compound which may be halogenated, although not particularly limited, for example, pentane (special flammable), hexane (primary petroleum), cyclohexane (primary petroleum), heptane (primary petroleum), octane (primary petroleum), nonane (secondary petroleum), decane (secondary petroleum) undecane (secondary petroleum), or dodecane (tertiary petroleum), 1,2-dichloroethylene (primary petroleum), 1-bromopropane (primary petroleum), 2-bromopropane (primary petroleum), iodoethane (secondary petroleum) 1,2-dichloroethane (primary petroleum), 1-bromo-1-propene (primary petroleum), 2-bromopropene (primary petroleum), 2,3-dibromopropene (tertiary petroleum), 1-chlorobutane (primary petroleum), 2-chlorobutane (primary petroleum), 2-chloro-2 methylpropane (primary petroleum), 1-bromobutane (primary petroleum), 2-bromobutane (secondary petroleum), 1-bromo-1-butene (primary petroleum), 2-bromo-2-butene (primary petroleum), 1-bromo-2-methylpropane (primary petroleum), 1-bromo-2-methylpropene (primary petroleum), 1-bromopentane (secondary petroleum), 2-bromopentane (primary petroleum), chlorocyclopentane (primary petroleum), chlorocyclohexane (secondary petroleum), or 3-chloro-1-butene (primary petroleum) may be mentioned. With respect to the halogenated hydrocarbon compound, from such a viewpoint that the merit of nonflammability is large, at least one of 1-bromo-1-butene (primary petroleum), 2-bromo-2-butene (primary petroleum), 1-bromo-2-methylpropane (primary petroleum), 1-bromo-2-methylpropene (primary petroleum), 1-bromopentane (secondary petroleum) and 2-bromopentane (primary petroleum) is preferred.

As the above-mentioned ether compound which may be halogenated, although not particularly limited, for example, diethyl ether (special flammable), tert-butyl methyl ether (primary petroleum), 2,3-dihydrofuran (primary petroleum), 2,5-dihydrofuran (primary petroleum), 1,3-dioxolane (primary petroleum), 2-methylfuran (primary petroleum), 2-chloroethyl methyl ether (primary petroleum), dichloromethyl methyl ether (secondary petroleum), 2-bromoethyl methyl ether (secondary petroleum), or tetrahydrofuran (primary petroleum) may be mentioned. With respect to the ether, from such a viewpoint that the merit of nonflammability is large, 2-chloroethyl methyl ether (primary petroleum), dichloromethyl methyl ether (secondary petroleum), or 2-bromoethyl methyl ether (secondary petroleum) is preferred.

As the above-mentioned ketone compound which may be halogenated, although not particularly limited, for example, dimethyl ketone (primary petroleum), methyl ethyl ketone (primary petroleum), diethyl ketone (primary petroleum), methyl propyl ketone (primary petroleum), or methyl isopropyl ketone (primary petroleum) may be mentioned.

As the above-mentioned ester compound which may be halogenated, although not particularly limited, for example, methyl formate ester (special flammable), ethyl formate ester (primary petroleum), propyl formate ester (primary petroleum), butyl formate ester (primary petroleum), methyl acetate ester (primary petroleum), ethyl acetate ester (primary petroleum), propyl acetate ester (primary petroleum), butyl acetate ester (secondary petroleum), methyl propionate ester (primary petroleum), ethyl propionate ester (primary petroleum), propyl propionate ester (secondary petroleum), or butyl propionate ester (secondary petroleum) may be mentioned.

As the above-mentioned epoxy compound which may be halogenated, although not particularly limited, for example, 1,2-butylene oxide (primary petroleum), 1,2-epoxypentane (primary petroleum), 1,2-epoxycyclopentane (primary petroleum), 3,4-epoxytetrahydrofuran (secondary petroleum), 1,2-epoxypentane (primary petroleum), 1,2-epoxycyclohexane (secondary petroleum), or 1,2-epoxy-4-vinylcyclohexane (secondary petroleum) may be mentioned, and from the viewpoint of easy availability, 1,2-butylene oxide (primary petroleum), 1,2-epoxypentane (primary petroleum), 1,2-epoxycyclopentane (primary petroleum), or 1,2-epoxy-4-vinylcyclohexane (secondary petroleum) is preferred.

As the above-mentioned amine compound which may be halogenated, although not particularly limited, for example, 2,2,6,6-tetramethylpiperidine or 1,2,2,6,6-pentamethyl-4-piperidyl methacrylate may be mentioned, and from the viewpoint of excellent stability effect, 1,2,2,6,6-pentamethyl-4-piperidyl methacrylate is preferred.

As the above-mentioned aromatic compound which may be halogenated, although not particularly limited, for example, benzene (primary petroleum), toluene (primary petroleum), ethylbenzene (secondary petroleum), cumene (secondary petroleum), styrene (secondary petroleum), anisole (secondary petroleum), benzaldehyde (secondary petroleum), xylene (secondary petroleum), chlorobenzene (secondary petroleum), bromobenzene (secondary petroleum), fluorobenzene (primary petroleum), o-difluorobenzene (primary petroleum), m-difluorobenzene (primary petroleum), p-difluorobenzene (primary petroleum), or trifluoromethylbenzene (primary petroleum) may be mentioned. With respect to the aromatic hydrocarbon, from such a viewpoint that the merit of nonflammability is large, trifluoromethylbenzene (primary petroleum) is preferred.

In the invention of the present application, with respect to the flammable liquid, from such a viewpoint that the flammability-reducing effect is excellent, a flammable liquid made of at least one type selected from the group consisting of 2,2,2-trifluoroethanol, ethanol, 70 vol% ethanol aqueous solution, 1-bromopropane, 1-bromo-2-methylpropane, 2-chloroethyl methyl ether, dichloromethyl methyl ether, toluene, 2-bromo-2-butene, 2-bromopentane, 2,3-dibromopropene, 1,2-dichloroethylene, decane, cyclohexane, 1,2-butylene oxide, 1,2-epoxypentane, 1,2-epoxycyclopentane, trifluoromethylbenzene, 1,2-epoxy-4-vinylcyclohexane, 2,2,6,6-tetramethylpyperidine and 1,2,2,6,6-pentamethyl-4-piperidyl methacrylate, is preferred (a mixture of two or more flammable liquids may be referred to also as a flammable liquid composition).

The present invention relates to a liquid composition comprising a flammable liquid and a fluoroiodoalkane represented by the above-mentioned formula (1), characterized in that the total amount of the above-mentioned flammable liquid and the above-mentioned fluoroiodoalkane is from 95 to 100 mass% of the entire amount of the above-mentioned liquid composition. The total amount of the above-mentioned flammable liquid and the above-mentioned fluoroiodoalkane is preferably from 97 to 100 mass%, more preferably from 99 to 100 mass%, of the entire amount of the above-mentioned liquid composition.

In the liquid composition of the present invention, the content of the flammable liquid is not particularly limited, but it is preferably from 60 to 99.9 wt%, more preferably from 70 to 99.9 wt%, further preferably from 80 to 99 wt%, still more preferably from 90 to 99 wt%, based on the entire amount of the liquid composition being 100 wt%.

In the liquid composition of the present invention, the content of the fluoroiodoalkane represented by the above-mentioned formula (1) is not particularly limited, but it is preferably from 0.1 to 50 wt%, more preferably from 0.1 to 40 wt%, further preferably from 0.1 to 30 wt%, still more preferably from 1 to 20 wt%, still further preferably from 1 to 10 wt%, based on the entire amount of the liquid composition being 100 wt%.

Due to its properties, the liquid composition of the present invention is expected to be utilized as a nonflammable cleaning agent.

With respect to the liquid composition of the present invention, from such a viewpoint that it is excellent in performance as a cleaning agent, the above-mentioned flammable liquid is preferably a flammable liquid containing at least 1-bromo-2-methylpropane and trifluoromethylbenzene, more preferably a flammable liquid containing at least 1-bromo-2-methylpropane, trifluoromethylbenzene and 1,2-epoxycyclopentane, further preferably a flammable liquid containing at least 1-bromo-2-methylpropane, trifluoromethylbenzene, 1,2-epoxycyclopentane and 1,2,2,6,6-pentamethyl-4-piperidyl methacrylate.

With respect to the liquid composition of the present invention, from such a viewpoint that it is excellent in performance as a cleaning agent, the content of the above-mentioned 1-bromo-2-methylpropane is preferably from 70 to 95 wt% of the entire amount of the above-mentioned liquid composition, more preferably from 75 to 92 wt% of the entire amount of the above-mentioned liquid composition, further preferably from 80 to 90 wt% of the entire amount of the above-mentioned liquid composition.

With respect to the liquid composition of the present invention, from such a viewpoint that it is excellent in performance as a cleaning agent, the content of trifluoromethylbenzene is preferably from 1 to 10 wt% of the entire amount of the above-mentioned liquid composition, more preferably from 2 to 9 wt% of the entire amount of the above-mentioned liquid composition, further preferably from 3 to 8 wt% of the entire amount of the above-mentioned liquid composition.

With respect to the liquid composition of the present invention, from such a viewpoint that it is excellent in performance as a cleaning agent, the content of 1,2-epoxycyclopentane is preferably from 0.5 to 5 wt% of the entire amount of the above-mentioned liquid composition, more preferably from 0.5 to 4 wt% of the entire amount of the above-mentioned liquid composition, further preferably from 1 to 3 wt% of the entire amount of the above-mentioned liquid composition.

With respect to the liquid composition of the present invention, from such a viewpoint that it is excellent in performance as a cleaning agent, the content of 1,2,2,6,6-pentamethyl-4-piperidyl methacrylate is preferably from 0.1 to 2 wt% of the entire amount of the above-mentioned liquid composition, more preferably from 0.1 to 1.8 wt% of the entire amount of the above-mentioned liquid composition, further preferably from 0.1 to 1.5 wt% of the entire amount of the above-mentioned liquid composition.

With respect to the liquid composition of the present invention, from such a viewpoint that it is excellent in performance as a cleaning agent, the content of the fluoroiodoalkane represented by the above-mentioned formula (1) is preferably from 1 to 20 wt% of the entire amount of the above-mentioned liquid composition, more preferably from 2 to 18 wt% of the entire amount of the above-mentioned liquid composition, further preferably from 5 to 15 wt% of the entire amount of the above-mentioned liquid composition.

That is, with respect to the liquid composition of the present invention, from such a viewpoint that it is excellent in performance as a cleaning agent, it is preferably one comprising 1,1,1-trifluoro-3-iodopropane, 1-bromo-2-methylpropane, trifluoromethylbenzene, 1,2-epoxycyclopentane, and 1,2,2,6,6-pentamethyl-4-piperidyl methacrylate, and, as their contents, it is preferably one comprising from 1 to 20 wt% of 1,1,1-trifluoro-3-iodopropane, from 70 to 95 wt% of 1-bromo-2-methylpropane, from 1 to 10 wt% of trifluoromethylbenzene, from 0.5 to 5 wt% of 1,2-epoxycyclopentane, and from 0.5 to 5 wt% of 1,2,2,6,6-pentamethyl-4-piperidyl methacrylate.

The liquid composition of the present invention may also contain a nonflammable liquid as a component other than the above-mentioned flammable liquid and the fluoroiodoalkane represented by the above-mentioned formula (1). As such a nonflammable liquid, although not particularly limited, for example, hexadecafluoro(1,3-dimethylcyclohexane), fluorocyclohexane, fluorocyclopentane, 1,1,2,2,3,3,4-heptafluorocyclopentane, methyl 1,1,2,2-tetrafluoroethyl ether, fluoromethyl 1,1,1,3,3,3-hexafluoroisopropyl ether, 2-chloro-1,1,2-trifluoroethyl methyl ether, ethyl 1,1,2,2-tetrafluoroethyl ether, 1,1,2,3,3,3-hexafluoropropyl methyl ether, 1,1,2,2-tetrafluoroethyl 2,2,2-trifluoroethyl ether, or 1-chloro-2,2,2-trifluoroethyl difluoromethyl ether may be mentioned. Further, with respect to such a nonflammable liquid, from the viewpoint of reducing the above-mentioned ODP and GWP, it is desirable to use as little amount as possible, and ultimately it is preferred that it is not contained in the liquid composition of the invention.

The liquid composition of the present invention may contain a component other than those listed above (e.g. a component that is solid at room temperature), and may contain, although not particularly limited, for example, the following component, which is commonly referred to as a stabilizer.

As such a stabilizer, although not particularly limited, for example, a benzophenone compound that is solid at room temperature, a nitrogen-containing compound that is solid at room temperature, or a phenol compound that is solid at room temperature, may be mentioned.

As the above-mentioned solid benzophenone compound, although not particularly limited, 2-hydroxy-4-methylbenzophenone (solid at room temperature), 2-hydroxy-4-methoxybenzophenone (solid at room temperature), 2-hydroxy-5-methylbenzophenone (solid at room temperature), or 2,2'-dihydroxybenzophenone (solid at room temperature) may be mentioned, and from such a viewpoint that the storage stability of the liquid composition of the present invention will be improved, 2-hydroxy-4-methylbenzophenone (solid at room temperature) is preferred.

As the above-mentioned nitrogen-containing compound at room temperature, although not particularly limited, 2,2,6,6-tetramethylpiperidine 1-oxy free radical (solid at room temperature), 2,2,6,6-tetramethyl-4-piperidyl methacrylate (solid at room temperature), or 4-glycidyloxy-2,2,6,6-tetramethylpiperidine 1-oxy free radical (solid at room temperature) may be mentioned.

As the above-mentioned phenol compound that is solid at room temperature, although not particularly limited, dibutylhydroxytoluene (solid at room temperature) may be mentioned.

In a case where the liquid composition of the present invention contains the above-mentioned stabilizer, its content is not particularly limited, but, based on the entire amount of the liquid composition being 100 wt%, it is preferably from 0.01 to 20 wt%, more preferably from 0.01 to 10 wt%, further preferably from 0.01 to 7 wt%, still more preferably from 0.01 to 3 wt%, still further preferably from 0.01 to 1 wt%.

In a case where the liquid composition of the present invention contains a component that is solid at room temperature such as the above-mentioned stabilizer, the component that is solid at room temperature, is usually present as dissolved in the above-mentioned liquid composition. This is because the concentration of the above-mentioned component that is solid at room temperature in the above-mentioned liquid composition is low.

The above-mentioned stabilizer can inhibit a compositional change due to decomposition (such as thermal, oxidative, or photolytic decomposition) of the above-mentioned flammable liquid composition or the above-mentioned fluoroiodoalkane.

The method of making a flammable liquid nonflammable of the present invention is accomplished by mixing the above-mentioned fluoroiodoalkane to the above-mentioned flammable liquid. As specifically shown in e.g. Examples, by adding and mixing the above-mentioned fluoroiodoalkane to the above-mentioned flammable liquid, the above-mentioned flammable liquid can lose its flash point and turns into a nonflammable liquid.

The liquid composition of the present invention has a characteristic of being nonflammable (also called non-ignition) and is expected to be utilized as a nonflammable liquid taking an advantage of the characteristic. The application of the liquid composition of the invention is not particularly limited, but may, for example, be a solvent, a washing agent, a refrigerant, a foaming agent, a fire extinguishing agent, or a disinfectant.

For example, the liquid composition of the present invention can be utilized as a washing agent to wash off contaminants such as oily components attached on various articles (articles to be washed).

The washing method of the present invention relates to a method characterized by bringing the above-mentioned liquid composition into contact with an article to be washed to wash off an attachment attached to the article to be washed.

In the washing method of the present invention, as an attachment to be washed off, an oily component may be mentioned, and as the oily component, although not particularly limited, for example, flux, cutting oil, quenching oil, rolling oil, lubricating oil, machine oil, press working oil, punching oil, drawing oil, assembly oil, wire drawing oil (collectively referred to also as processing oil), mold release agent, wax, ink, or oily stain containing dust may be mentioned. The washing composition of the present invention is excellent in solubility of an oily component such as processing oil, and thus is preferably used for washing an attachment including the oily component.

Further, as the above-mentioned article to be washed, although not particularly limited, an article which may be made of a various material, such as metal, plastic, elastomer, glass, ceramic, composite material, natural fiber, synthetic fiber, cloth, or woven fabric may be mentioned. As more specific examples of the article to be washed, although not particularly limited, for example, textile products, medical instruments, precision machinery, optical machinery, or parts thereof may be mentioned. As specific examples of the textile products, medical instruments, precision machinery, optical machinery, or parts thereof, although not particularly limited, for example, ICs, capacitors, printed circuit boards, micro motors, relays, bearings, optical lenses, or glass substrates, may be mentioned.

In the washing method of the present invention, if the feature of contacting the washing composition of the present invention with an article to be washed is satisfied, other conditions, etc., are not particularly limited. By the contact, it is possible to remove a contaminant attached to the surface of the article to be washed.

As the specific washing method by the contact, although not particularly limited, for example, hand-wiping, immersion washing, spray washing, immersion boiling washing, immersion oscillating washing, immersion ultrasonic washing, steam washing, or a method having these combined, may be employed. In such a washing method, washing conditions such as the contact time, the number of contact times, the temperature of the washing composition of the present invention at that time, etc. may be suitably selected depending on the washing method. Further, as the washing device, a known one may be suitably selected depending on each washing method. By such contact, it is possible to wash off an attachment attached to the article to be washed.

The washing method of the present invention is not particularly limited, but it is preferred, for example, to make it to be a washing method comprising a contact step of contacting an article to be washed, with a washing composition in a liquid phase, and a steam contact step, after the contact step, of evaporating the washing composition and exposing the article to be washed, with the generated steam, whereby the cleanliness of the article to be washed can be improved.

With respect to the liquid composition of the present invention, as mentioned above, it may be used as a disinfectant. That is, the disinfectant of the present invention consists of the above-mentioned liquid composition, and is widely used in various applications for the purpose of eliminating microorganisms. For example, it may be used for disinfecting the outer skin, as a bactericidal disinfectant, hemorrhoid medicine, athlete's foot medicine, cleaning cotton, bad breath prevention, acne prevention, armpit odor prevention, hand wipes, skin wipes, etc., and for disinfecting utensils, for refrigerators, toilet seats, tableware, toys, houses, etc. The disinfectant is expected to be utilized in fields where conventional flammable disinfectants are difficult to be used, taking advantage of its nonflammable and non-ignition characteristics.

### EXAMPLES

In the following, the present invention will be described in detail with reference to Examples. However, the present invention is not to be interpreted as limited by these Examples.

### [Method for evaluating nonflammability]

In evaluating the nonflammability of the liquid composition of the present invention, measurements were conducted in accordance with ASTM D6450 by using the MINIFLASH FP VISION manufactured by Grabner.

### [Method for measuring flash point]

In measuring the flash point of the liquid composition of the present invention, the flash point was measured by conducting tag-sealed and Cleveland open flash point measurements in accordance with the method described in JIS K2265.

### [Method for evaluating washing properties]

In evaluating the washing properties of the liquid composition of the present invention, the removal rate of processing oil attached to the article to be washed was measured by using the compact washing device F-100M manufactured by Shin-Otsuka Co, Ltd. As the article to be washed, a commercially available SUS bolt was used; as the processing oil, SUGICUT C-168AS manufactured by Sugimura Chemical Industrial Co., Ltd. was used; and UV-2600i manufactured by Shimadzu Corporation was used to measure the removal rate.

The washing time was 4 minutes (boiling: 1 minute, ultrasonic waves: 1 minute, steam: 1 minute, drying: 1 minute). To the article to be washed, after the washing, by HC-UV45 manufactured by Tosoh Corporation, ultrasonic waves were applied to extract the processing oil remained on the article to be washed. By measuring the UV of the extracted liquid, the removal rate of processing oil was calculated.

### Example 1-1

A composition having an optional amount of 1,1,1-trifluoro-2-iodoethane mixed to 2,2,2-trifluoroethanol was prepared. Then, the composition which becomes to be nonflammable was confirmed by using MINIFLASH FP VISION manufactured by Grabner. As a result, when 2,2,2-trifluoroethanol and 1,1,1-trifluoro-2-iodoethane were combined to make 100 wt%, all liquid compositions containing at least 1 wt% of 1,1,1-trifluoro-2-iodoethane showed nonflammability.

### Example 1-2

In Example 1-1, except that 1,1,1-trifluoro-3-iodopropane was used instead of 1,1, 1-trifluoro-2-iodoethane, the same operation as in Example 1-1 was carried out, and the composition which becomes to be nonflammable was confirmed. As a result, when 2,2,2-trifluoroethanol and 1,1,1-trifluoro-3-iodopropane were combined to make 100 wt%, all liquid compositions containing at least 1 wt% of 1,1,1-trifluoro-3-iodopropane showed nonflammability.

### Example 1-3

In Example 1-1, except that a 70 vol% ethanol aqueous solution was used instead of 2,2,2-trifluoroethanol, the same operation as in Example 1-1 was carried out, and the composition which becomes to be nonflammable was confirmed. As a result, when the 70 vol% ethanol aqueous solution and 1,1,1-trifluoro-2-iodoethane were combined to make 100 wt%, all liquid compositions containing at least 10 wt% of 1,1,1-trifluoro-2-iodoethane showed nonflammability.

### Example 1-4

In Example 1-3, except that 1,1,1-trifluoro-3-iodopropane was used instead of 1,1,1-trifluoro-2-iodoethane, the same operation as in Example 1-3 was carried out, and the composition which becomes to be nonflammable was confirmed. As a result, when a 70 vol% ethanol aqueous solution and 1,1,1-trifluoro-3-iodopropane were combined to make 100 wt%, all liquid compositions containing at least 20 wt% by weight of 1,1,1-trifluoro-3-iodopropane showed nonflammability.

### Example 1-5

In Example 1-1, except that 1-bromo-2-methylpropane was used instead of 2,2,2-trifluoroethanol, the same operation as in Example 1-1 was carried out, and the composition which becomes to be nonflammable was confirmed. As a result, when 1-bromo-2-methylpropane and 1,1,1-trifluoro-2-iodoethane were combined to make 100 wt%, all liquid compositions containing at least 5 wt% of 1,1,1-trifluoro-2-iodoethane showed nonflammability.

### Example 1-6

In Example 1-5, except that 1,1,1-trifluoro-3-iodopropane was used instead of 1,1, 1-trifluoro-2-iodoethane, the same operation as in Example 1-5 was carried out, and the composition which becomes to be nonflammable was confirmed. As a result, when 1-bromo-2-methylpropane and 1,1,1-trifluoro-3-iodopropane were combined to make 100 wt%, all liquid compositions containing at least 5 wt% of 1,1,1-trifluoro-3-iodopropane showed nonflammability.

### Example 1-7

In Example 1-1, except that 2-chloroethyl methyl ether was used instead of 2,2,2-trifluoroethanol, the same operation as in Example 1-1 was carried out, and the composition which becomes to be nonflammable was confirmed. As a result, when 2-chloroethyl methyl ether and 1,1,1-trifluoro-2-iodoethane were combined to make 100 wt%, all liquid compositions containing at least 20 wt% of 1,1,1-trifluoro-2-iodoethane showed nonflammability.

### Example 1-8

In Example 1-7, except that 1,1,1-trifluoro-3-iodopropane was used instead of 1,1,1-trifluoro-2-iodoethane, the same operation as in Example 1-7 was carried out, and the composition which becomes to be nonflammable was confirmed. As a result, when 2-chloroethyl methyl ether and 1,1,1-trifluoro-3-iodopropane were combined to make 100 wt%, all liquid compositions containing at least 20 wt% of 1,1,1-trifluoro-3-iodopropane showed nonflammability.

### Example 1-9

In Example 1-1, except that dichloromethyl methyl ether was used instead of 2,2,2-trifluoroethanol, the same operation as in Example 1-1 was carried out, and the composition which becomes to be nonflammable was confirmed. As a result, when dichloromethyl methyl ether and 1,1,1-trifluoro-2-iodoethane were combined to make 100 wt%, all liquid compositions containing at least 1 wt% of 1,1,1-trifluoro-2-iodoethane showed nonflammability.

### Example 1-10

In Example 1-9, except that 1,1,1-trifluoro-3-iodopropane was used instead of 1,1, 1-trifluoro-2-iodoethane, the same operation as in Example 1-9 was carried out, and the composition which becomes to be nonflammable was confirmed. As a result, when dichloromethyl methyl ether and 1,1,1-trifluoro-3-iodopropane were combined to make 100 wt%, all liquid compositions containing at least 1 wt% of 1,1,1-trifluoro-3-iodopropane showed nonflammability.

### Example 1-11

In Example 1-1, except that 1,1,1-trifluoro-4-iodobutane was used instead of 1,1, 1-trifluoro-2-iodoethane, the same operation as in Example 1-1 was carried out, and the composition which becomes to be nonflammable was confirmed. When 2,2,2-trifluoroethanol and 1,1,1-trifluoro-4-iodobutane were combined to make 100 wt%, all liquid compositions containing at least 1 wt% of 1,1,1-trifluoro-4-iodobutane showed nonflammability.

### Example 1-12

In Example 1-3, except that 1,1,1-trifluoro-4-iodobutane was used instead of 1,1,1-trifluoro-2-iodoethane, the same operation as in Example 1-3 was carried out, and the composition which becomes to be nonflammable was confirmed. When a 70 vol% ethanol aqueous solution and 1,1,1-trifluoro-4-iodobutane were combined to make 100 wt%, all liquid compositions containing at least 20 wt% of 1,1,1-trifluoro-4-iodobutane showed nonflammability.

### Example 1-13

In Example 1-5, except that 1,1,1-trifluoro-4-iodobutane was used instead of 1,1,1-trifluoro-2-iodoethane, the same operation as in Example 1-5 was carried out, and the composition which becomes to be nonflammable was confirmed. When 1-bromo-2-methylpropane and 1,1,1-trifluoro-4-iodobutane were combined to make 100 wt%, all liquid compositions containing at least 5 wt% of 1,1,1-trifluoro-4-iodobutane showed nonflammability.

### Example 1-14

In Example 1-7, except that 1,1,1-trifluoro-4-iodobutane was used instead of 1,1,1-trifluoro-2-iodoethane, the same operation as in Example 1-7 was carried out, and the composition which becomes to be nonflammable was confirmed. When 2-chloroethyl methyl ether and 1,1,1-trifluoro-4-iodobutane were combined to make 100 wt%, all liquid compositions containing at least 20 wt% of 1,1,1-trifluoro-4-iodobutane showed nonflammability.

### Example 1-15

In Example 1-9, except that 1,1,1-trifluoro-4-iodobutane was used instead of 1,1,1-trifluoro-2-iodoethane, the same operation as in Example 1-9 was carried out, and the composition which becomes to be nonflammable was confirmed. When dichloromethyl methyl ether and 1,1,1-trifluoro-4-iodobutane were combined to make 100 wt%, all liquid compositions containing at least 1 wt% of 1,1,1-trifluoro-4-iodobutane showed nonflammability.

### Example 1-16

In Example 1-1, except that toluene was used instead of 2,2,2-trifluoroethanol, the same operations as in Example 1-1 was carried out, and the composition which becomes to be nonflammable was confirmed. When toluene and 1,1,1-trifluoro-2-iodoethane were combined to make 100 wt%, all liquid compositions containing at least 20 wt% of 1,1,1-trifluoro-2-iodoethane showed nonflammability.

### Example 1-17

In Example 1-16, except that 1,1,1-trifluoro-3-iodopropane was used instead of 1,1,1-trifluoro-2-iodoethane, the same operation as in Example 1-16 was carried out, and the composition which becomes to be nonflammable was confirmed. When toluene and 1,1,1-trifluoro-3-iodopropane were combined to make 100 wt%, all liquid compositions containing at least 20 wt% of 1,1,1-trifluoro-3-iodopropane showed nonflammability.

### Example 1-18

In Example 1-1, except that 2-bromo-2-butene was used instead of 2,2,2-trifluoroethanol, the same operation as in Example 1-1 was carried out, and the composition which becomes to be nonflammable was confirmed. When 2-bromo-2-butene and 1,1,1-trifluoro-2-iodoethane were combined to make 100 wt%, all liquid compositions containing at least 10 wt% of 1,1,1-trifluoro-2-iodoethane showed nonflammability.

### Example 1-19

In Example 1-18, except that 1,1,1-trifluoro-3-iodopropane was used instead of 1,1,1-trifluoro-2-iodoethane, the same operation as in Example 1-18 was carried out, and the composition which becomes to be nonflammable was confirmed. When 2-bromo-2-butene and 1,1,1-trifluoro-3-iodopropane are combined to make 100 wt%, all liquid compositions containing at least 10 wt% of 1,1,1-trifluoro-3-iodopropane showed nonflammability.

### Example 1-20

In Example 1-1, except that 2-bromopentane was used instead of 2,2,2-trifluoroethanol, the same operation as in Example 1-1 was carried out, and the composition which becomes to be nonflammable was confirmed. When 2-bromopentane and 1,1,1-trifluoro-2-iodoethane were combined to make 100 wt%, all liquid compositions containing at least 1 wt% of 1,1,1-trifluoro-2-iodoethane showed nonflammability.

### Example 1-21

In Example 1-20, except that 1,1,1-trifluoro-3-iodopropane was used instead of 1,1, 1-trifluoro-2-iodoethane, the same operation as in Example 1-20 was carried out, and the composition which becomes to be nonflammable was confirmed. When toluene and 1,1,1-trifluoro-3-iodopropane were combined to make 100 wt%, all liquid compositions containing at least 10 wt% of 1,1,1-trifluoro-3-iodopropane showed nonflammability.

### Example 1-22

In Example 1-1, except that 2,3-dibromopropene was used instead of 2,2,2-trifluoroethanol, the same operation as in Example 1-1 was carried out, and the composition which becomes to be nonflammable was confirmed. When 2,3-dibromopropene and 1,1,1-trifluoro-2-iodoethane were combined to make 100 wt% all liquid compositions containing at least 1 wt% of 1,1,1-trifluoro-2-iodoethane showed nonflammability.

### Example 1-23

In Example 1-22, except that 1,1,1-trifluoro-3-iodopropane was used instead of 1,1,1-trifluoro-2-iodoethane, the same operation as in Example 1-22 was carried out, and the composition which becomes to be nonflammable was confirmed. When 2,3-dibromopropene and 1,1,1-trifluoro-3-iodopropane were combined to make 100 wt%, all liquid compositions containing at least 1 wt% of 1,1,1-trifluoro-3-iodopropane showed nonflammability.

### Example 1-24

In Example 1-1, except that a flammable composition (composition 1) with a weight ratio of 1-bromo-2-methylpropane and 1,2-butylene oxide being 9:1 was used instead of 2,2,2-trifluoroethanol, the same operation as in Example 1-1 was carried out, and the composition which becomes to be nonflammable was confirmed. When composition 1 and 1,1,1-trifluoro-2-iodoethane were combined to make 100 wt%, all liquid compositions containing at least 20 wt% of 1,1,1-trifluoro-2-iodoethane showed nonflammability.

### Example 1-25

In Example 1-24, except that 1,1,1-trifluoro-3-iodopropane was used instead of 1,1,1-trifluoro-2-iodoethane, the same operation as in Example 1-24 was carried out, and the composition which becomes to be nonflammable was confirmed. When composition 1 and 1,1,1-trifluoro-3-iodopropane were combined to make 100 wt%, all liquid compositions containing at least 20 wt% of 1,1,1-trifluoro-3-iodopropane showed nonflammability.

### Example 1-26

In Example 1-1, except that a flammable composition (composition 2) with a weight ratio of 1-bromo-2-methylpropane and 1,2-epoxycyclopentane being 9:1 was used instead of 2,2,2-trifluoroethanol, the same operation as in Example 1-1 was carried out, and the composition which becomes to be nonflammable, was confirmed. When composition 2 and 1,1,1-trifluoro-2-iodoethane were combined to make 100 wt%, all liquid compositions containing at least 5 wt% of 1,1,1-trifluoro-2-iodoethane showed nonflammability.

### Example 1-27

In Example 1-26, except that 1,1,1-trifluoro-3-iodopropane was used instead of 1,1,1-trifluoro-2-iodoethane, the same operation as in Example 1-26 was carried out, and the composition which becomes to be nonflammable, was confirmed. When composition 2 and 1,1,1-trifluoro-3-iodopropane were combined to make 100 wt%, all liquid compositions containing at least 5 wt% of 1,1,1-trifluoro-3-iodopropane showed nonflammability.

### Example 1-28

In Example 1-1, except that a flammable composition (composition 3) with a weight ratio of 1-bromo-2-methylpropane and trifluoromethylbenzene being 89:11 was used instead of 2,2,2-trifluoroethanol, and1,1,1-trifluoro-3-iodopropane was used instead of 1,1,1-trifluoro-2-iodoethane, the same operation as in Example 1-1 was carried out, and the composition which becomes to be nonflammable, was confirmed. When composition 3 and 1,1,1-trifluoro-3-iodopropane were combined to make 100 wt%, all liquid compositions containing at least 5 wt% of 1,1,1-trifluoro-3-iodopropane showed nonflammability.

### Example 1-29

In Example 1-1, except that a flammable composition (composition 4) with a weight ratio of 1-bromo-2-methylpropane and 1,2-epoxy-4-vinylcyclohexane being 9:1 was used instead of 2,2,2-trifluoroethanol, the same operation as in Example 1-1 was carried out, and the composition which becomes to be nonflammable, was confirmed. When composition 4 and 1,1,1-trifluoro-2-iodoethane were combined to make 100 wt%, all liquid compositions containing at least 1 wt% of 1,1,1-trifluoro-2-iodoethane showed nonflammability.

### Example 1-30

In Example 1-2, except that a flammable composition (composition 5) with a weight ratio of 1-bromo-2-methylpropane, trifluoromethylbenzene and 1,2-epoxycyclopentane being 94:5:1 was used instead of 2,2,2-trifluoroethanol, the same operation as in Example 1-2 was carried out, and the composition which becomes to be nonflammable, was confirmed. When composition 5 and 1,1,1-trifluoro-3-iodopropane were combined to make 100 wt%, all liquid compositions containing at least 5 wt% of 1,1,1-trifluoro-3-iodopropane showed nonflammability.

### Example 1-31

In Example 1-2, except that a flammable composition (composition 6) with a weight ratio of 1-bromo-2-methylpropane, trifluoromethylbenzene, 1,2-epoxycyclopentane, 1,2,2,6,6-pentamethyl-4-piperidyl methacrylate and 4-glycidyloxy-2,2,6,6- tetramethylpiperidine 1-oxyfree radicals being 93:5:1:0.5:0.01, was used instead of 2,2,2-trifluoroethanol, the same operation as in Example 1-2 was carried out, and the composition which becomes to be nonflammable, was confirmed. When composition 6 and 1,1,1-trifluoro-3-iodopropane were combined to make 100 wt%, all liquid compositions containing at least 5 wt% of 1,1,1-trifluoro-3-iodopropane showed nonflammability.

### Example 1-32

In Example 1-2, except that a flammable composition (composition 7) with a weight ratio of 1-bromo-2-methylpropane and 1,2-epoxypentane being 97:3, was used instead of 2,2,2-trifluoroethanol, the same operation as in Example 1-2 was carried out, and the composition which becomes to be nonflammable, was confirmed. When composition 7 and 1,1,1-trifluoro-3-iodopropane were combined to make 100 wt%, all liquid compositions containing at least 10 wt% of 1,1,1-trifluoro-3-iodopropane showed nonflammability.

### Comparative Example 1-1

A composition having an optional amount of Novec^{™} 7200 (a fluoroether compound represented by C₄F₉OC₂H₅, manufactured by 3M) mixed to a 70 vol% ethanol aqueous solution, was prepared. Then, the composition which becomes to be nonflammable, was confirmed by using MINIFLASH FP VISION manufactured by Grabner. As a result, when the 70 vol% ethanol aqueous solution and Novec^{™} 7200 were combined to make 100 wt%, all liquid compositions containing at least 40 wt% of Novec^{™} 7200 showed nonflammability. Here, Novec is a registered trademark of 3M Company.

### Comparative Example 1-2

In Comparative Example 1-1, except that toluene was used instead of the 70 vol% ethanol aqueous solution, the same operation as in Comparative Example 1-1 was carried out, and the composition which becomes to be nonflammable, was confirmed. As a result, when toluene and Novec^{™} 7200 were combined to make 100 wt%, all liquid compositions containing at least 60 wt% of Novec^{™} 7200 showed nonflammability.

### Comparative Example 1-3

In Comparative Example 1-2, except that CELEFIN 1233Z (chlorofluoroalkene compound represented by CF₃CH=CHCl, manufactured by Central Glass Co., Ltd.) was used instead of Novec 7200 (manufactured by 3M), the same operation as in Comparative Example 1-2 was carried out, and the composition which becomes to be nonflammable, was confirmed. As a result, when toluene and CELEFIN 1233Z were combined to make 100 wt%, all liquid compositions containing at least 40 wt% of CELEFIN 1233Z showed nonflammability. Here, CELEFIN is a registered trademark of Central Glass Co., Ltd.

### Comparative Example 1-4

In Comparative Example 1-1, except that nonafluorobutyl iodide (C₄F₉I) was used instead of Novec^{™} 7200, the same operation as in Comparative Example 1-1 was carried out, and the composition which becomes to be nonflammable, was confirmed. As a result, when a 70 vol% ethanol aqueous solution and nonafluorobutyl iodide were combined to make 100 wt%, the composition showed flammability even when 50 wt% of nonafluorobutyl iodide was added.

### Comparative Example 1-5

In Comparative Example 1-1, except that undecafluoropentyl iodide (C₅F₁₁I) was used instead of Novec^{™} 7200, the same operation as in Comparative Example 1-1 was carried out, and the composition which becomes to be nonflammable, was confirmed. As a result, when a 70 vol% ethanol aqueous solution and undecafluoropentyl iodide were combined to make 100 wt%, the composition showed flammability even when 50 wt% of undecafluoropentyl iodide was added.

### Comparative Example 1-6

In Comparative Example 1-1, except that tridecafluorohexyl iodide (C₆ F₁₃ I) was used instead of Novec^{™} 7200, the same operation as in Comparative Example 1-1 was carried out, and the composition which becomes to be nonflammable, was confirmed. As a result, when a 70 vol% ethanol aqueous solution and tridecafluorohexyl iodide were combined to make 100 wt%, the composition showed flammability even when 50 wt% of tridecafluorohexyl iodide was added.

### Comparative Example 1-7

In Comparative Example 1-4, except that toluene was used instead of 70 vol% ethanol aqueous solution, the same operation as in Comparative Example 1-4 was carried out, and the composition which becomes to be nonflammable, was confirmed. As a result, when toluene and nonafluorobutyl iodide were combined to make 100 wt%, the composition showed flammability even when 50 wt% of nonafluorobutyl iodide was added.

### Comparative Example 1-8

In Comparative Example 1-5, except that toluene was used instead of 70 vol% ethanol aqueous solution, the same operation as in Comparative Example 1-5 was carried out, and the composition which becomes to be nonflammable, was confirmed. As a result, when toluene and undecafluoropentyl iodide were combined to make 100 wt%, the composition showed flammability even when 50 wt% of undecafluoropentyl iodide was added.

### Comparative Example 1-9

In Comparative Example 1-6, except that toluene was used instead of 70 vol% ethanol aqueous solution, the same operation as in Comparative Example 1-6 was carried out, and the composition which becomes to be nonflammable, was confirmed. As a result, when a 70 vol% ethanol aqueous solution and tridecafluorohexyl iodide were combined to make 100 wt%, the composition showed flammability even when 50 wt% of tridecafluorohexyl iodide was added.

The results of the foregoing are summarized in Tables 1 and 2.

**[Table 1]**

| Flammable solvent | Minimum content of fluoroiodoalkane showing nonflammability | | |
|---|---|---|---|
| | 1 ,1 ,1-trifluolo-2-iodoethane | 1,1 ,1-trifluoro-3-iodopropane | 1,1,1-trifluoro-4-iodobutane |
| 2,2,2-trifluoroethanol | 1 wt% (Example 1-1) | 1 wt% (Example 1-2) | 1 wt% (Example 1-11) |
| 70 vol% ethanol aqueous solution | 10 wt% (Example 1-3) | 20 wt% (Example 1-4) | 20 wt% (Example 1-12) |
| 1-bromo-2-methyl propane | 5 wt% (Example 1-5) | 5 wt% (Example 1-6) | 5 wt% (Example 1-13) |
| 2-chloroethyl methyl ether | 20 wt% (Example 1-7) | 20 wt% (Example 1-8) | 20 wt% (Example 1-14) |
| Dichloromethyl methyl ether | 1 wt% (Example 1-9) | 1 wt% (Example 1-10) | 1 wt% (Example 1-15) |
| Toluene | 20 wt% (Example 1-16) | 20 wt% (Example 1-17) | - |
| 2-bromo-2-butene | 10 wt% (Example 1-18) | 10 wt% (Example 1-19) | - |
| 2-bromopentane | 1 wt% (Example 1-20) | 10 wt% (Example 1-21) | - |
| 2,3-dibromopropene | 1 wt% (Example 1-22) | 1 wt% (Example 1-23) | - |
| Composition 1 | 20 wt% (Example 1-24) | 20 wt% (Example 1-25) | - |
| Composition 2 | 5 wt% (Example 1-26) | 5 wt% (Example 1-27) | - |
| Composition 3 | - | 5 wt% (Example 1-28) | - |
| Composition 4 | 1 wt% (Example 1-29) | - | - |
| Composition 5 | - | 5 wt% (Example 1-30) | - |
| Composition 6 | - | 5 wt% (Example 1-31) | - |
| Composition 7 | - | 10 wt% (Example 1-32) | - |

**[Table 2]**

| Flammable solvent | Minimum content of nonflammable solvent showing nonflammability | | | | |
|---|---|---|---|---|---|
| | Novec^{™} 7200 | CELEFIN^{®} 1233Z | Nonafluoro butyliodide | Undecafluoro butyliodide | Tridecafluoro butyliodide |
| 70 vol% ethanol aqueous solution | 40 wt% (Comp. Ex. 1-1) | - | >50 wt% (Comp. Ex. 1-4) | >50 wt% (Comp. Ex. 1-5) | >50 wt% (Comp. Ex. 1-6) |
| Toluene | 60 wt% (Comp. Ex. 1-2) | 40 wt% (Comp. Ex. 1-3) | >50 wt% (Comp. Ex. 1-7) | >50 wt% (Comp. Ex. 1-8) | >50 wt% (Comp. Ex. 1-9) |

The liquid composition of the present invention is one which achieves the effect of showing nonflammability by containing a fluoroiodoalkane represented by the above-mentioned formula (1), but, as described in Table 1, has a characteristic of showing a high nonflammability effect even with a small content as compared with conventionally known nonflammable materials. Therefore, the liquid composition of the present invention is one which has the effect of making the flammable liquid to be nonflammable while maintaining the original characteristics of the flammable liquid.

### Example 2-1

The flash point of a liquid composition with a weight ratio of 1,1,1-trifluoro-2-iodoethane and 2,2,2-trifluoroethanol being 1 wt%:99 wt% was measured. As a result, it was a nonflammable liquid.

### Example 2-2

The flash point of a liquid composition with a weight ratio of 1,1,1-trifluoro-2-iodoethane and 70 vol% ethanol aqueous solution being 10 wt%:90 wt% was measured. As a result, it was a nonflammable liquid.

### Example 2-3

The flash point of a liquid composition with a weight ratio of 1,1,1-trifluoro-2-iodoethane and 1-bromo-2-methylpropane being 5 wt%:95 wt% was measured. As a result, it was a nonflammable liquid.

### Example 2-4

The flash point of a liquid composition with a weight ratio of 1,1,1-trifluoro-3-iodopropane and 2,2,2-trifluoroethanol being 1 wt%:99 wt% was measured. As a result, it was a nonflammable liquid.

### Example 2-5

The flash point of a liquid composition with a weight ratio of 1,1,1-trifluoro-3-iodopropane and 1-bromo-2-methylpropane being 5 wt%:95 wt% was measured. As a result, it was a nonflammable liquid.

### Example 2-6

The flash point of a liquid composition with a weight ratio of 1,1,1-trifluoro-3-iodopropane, 1-bromo-2-methylpropane and 1,2-epoxycyclopentane being 10 wt%:87 wt%:3 wt% was measured. As a result, it was a nonflammable liquid.

### Example 2-7

The flash point of a liquid composition with a weight ratio of 1,1,1-trifluoro-3-iodopropane, 1-bromo-2-methylpropane and 1,2-epoxycyclopentane being 5 wt%:94 wt%:1 wt% was measured. As a result, it was a nonflammable liquid.

### Example 2-8

The flash point of a liquid composition with a weight ratio of 1,1, 1-trifluoro-3-iodopropane and 1-bromopentane being 10 wt%:90 wt% was measured. As a result, it was a nonflammable liquid.

### Example 2-9

The flash point of a liquid composition with a weight ratio of 1,1,1-trifluoro-3-iodopropane, 1-bromo-2-methylpropane and 1,2-epoxy-4-vinylcyclohexane being 20 wt%:79 wt%:1 wt% was measured. As a result, it was a nonflammable liquid.

### Example 2-10

The flash point of a liquid composition with a weight ratio of 1,1,1-trifluoro-3-iodopropane, 1-bromo-2-methylpropane and trifluoromethylbenzene being 5 wt%:85 wt%:10 wt% was measured. As a result, it was a nonflammable liquid.

### Example 2-11

The flash point of a liquid composition with a weight ratio of 1,1,1-trifluoro-3-iodopropane, 1-bromo-2-methylpropane, trifluoromethylbenzene, 1,2 epoxycyclopentane, 1,2,2,6,6-pentamethyl-4-piperidyl methacrylate and 4-glycidyloxy-2,2,6,6-tetramethylpiperidine being 4.9 wt%:88.7 wt%:4.9 wt%:1.0 wt%:0.5 wt%:0.005 wt% was measured. As a result, it was a nonflammable liquid.

### Example 3-1

Using the liquid composition described in Example 2-3, washability was evaluated. As a result, the oil removal rate was at least 99%.

### Example 3-2

Using the liquid composition described in Example 2-5, washability was evaluated. As a result, the oil removal rate was at least 99%.

### Example 3-3

Using the liquid composition described in Example 2-6, washability was evaluated. As a result, the oil removal rate was at least 99%.

### Example 3-4

Using the liquid composition described in Example 2-7, washability was evaluated. As a result, the oil removal rate was at least 99%.

### Example 3-5

Using the liquid composition described in Example 2-9, washability was evaluated. As a result, the oil removal rate was at least 99%.

### Example 3-6

Using the liquid composition described in Example 2-9, washability was evaluated. As a result, the oil removal rate was at least 99%.

### Example 3-7

Using the liquid composition described in Example 2-10, washability was evaluated. As a result, the oil removal rate was at least 99%.

### Example 3-8

Using the liquid composition described in Example 2-11, washability was evaluated. As a result, the oil removal rate was at least 99%.

### Comparative Example 3-1

Using nonafluorobutyl iodide (C₄F₉I), washability was evaluated. As a result, the oil removal rate was 10%. Further, corrosion of a bolt made of SUS, which was the item to be washed, was confirmed. No such corrosion was observed in Examples 3-1 to 3-8.

The present invention has been described in detail and with reference to specific embodiments, but it is apparent to those skilled in the art that various changes and modifications can be made without departing from the essence and scope of the present invention.

The entire contents of the claims, drawings and abstract of Japanese Patent Application No. 2021-009596, filed on January 25, 2021, are hereby cited and incorporated as the disclosure of the specification of the invention.

### INDUSTRIAL APPLICABILITY

Conventional flammable solvents, refrigerants, foaming agents, extinguishing agents or disinfectants can be efficiently and easily made to be nonflammable, and it is possible to provide solvents, refrigerants, foaming agents, extinguishing agents or disinfectants having a risk of flammability or fire reduced.

## Claims

1. A liquid composition comprising a flammable liquid and a fluoroiodoalkane represented by the following formula (1): 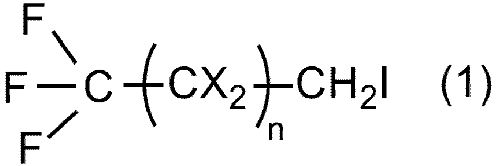 (in the formula, X each independently represents H or F, and n represents an integer of from 0 to 5), **characterized in that** the total amount of the above-mentioned flammable liquid and the above-mentioned fluoroiodoalkane is from 95 to 100 mass% of the entire amount of the above-mentioned liquid composition.

2. The liquid composition according to Claim 1, wherein the fluoroiodoalkane represented by the above-mentioned formula (1) is 1,1,1-trifluoro-2-iodoethane, 1,1,1-trifluoro-3- iodopropane, or 1,1,1-trifluoro-4-iodobutane.

3. The liquid composition according to Claim 1 or 2, wherein the above-mentioned flammable liquid is a flammable liquid made of at least one type selected from the group consisting of an alcohol compound which may be halogenated, a hydrocarbon compound which may be halogenated, an ether compound which may be halogenated, an epoxy compound which may be halogenated, an amine compound which may be halogenated, and an aromatic compound which may be halogenated.

4. The liquid composition according to Claim 1 or 2, wherein the above-mentioned flammable liquid is a flammable liquid made of at least one type selected from the group consisting of 2,2,2-trifluoroethanol, ethanol, a 70 vol% ethanol aqueous solution, 1-bromopropane, 1-bromo-2-methylpropane, 2-chloroethyl methyl ether, dichloromethyl methyl ether, toluene, 2-bromo-2-butene, 2-bromopentane, 2,3-dibromopropene, 1,2-dichloroethylene, decane, cyclohexane, 1,2-butylene oxide, 1,2-epoxypentane, 1,2-epoxycyclopentane, trifluoromethylbenzene, 1,2-epoxy-4-vinylcyclohexane, 2,2,6,6-tetramethylpiperidine, and 1,2,2,6,6-pentamethyl-4-piperidyl methacrylate.

5. The liquid composition according to any one of Claims 1 to 4, wherein in the total amount of the above-mentioned flammable liquid and fluoroiodoalkane, the proportion of the above-mentioned fluoroiodoalkane is from 0.1 to 50 mass%.

6. The liquid composition according to any one of Claims 1 to 5, wherein the above-mentioned flammable liquid is a flammable liquid containing at least 1-bromo-2-methylpropane and trifluoromethylbenzene.

7. The liquid composition according to any one of Claims 1 to 6, wherein the above-mentioned flammable liquid is a flammable liquid containing at least 1-bromo-2-methylpropane, trifluoromethylbenzene and 1,2-epoxycyclopentane.

8. The liquid composition according to any one of Claims 1 to 6, wherein the above-mentioned flammable liquid is a flammable liquid containing at least 1-bromo-2-methylpropane, trifluoromethylbenzene, 1,2-epoxycyclopentane and 1,2,2,6,6-pentamethyl-4-piperidyl methacrylate.

9. The liquid composition according to any one of Claims 5 to 8, wherein the content of the above-mentioned 1-bromo-2-methylpropane is from 70 to 95 wt% of the entire amount of the above-mentioned liquid composition.

10. The liquid composition according to any one of Claims 5 to 8, wherein the content of the above-mentioned trifluoromethylbenzene is from 1 to 10 wt% of the entire amount of the above-mentioned liquid composition.

11. The liquid composition according to Claim 7 or 8, wherein the content of the above-mentioned 1,2-epoxycyclopentane is from 0.5 to 5 wt% of the entire amount of the above-mentioned liquid composition.

12. The liquid composition according to Claim 8, wherein the content of the above-mentioned 1,2,2,6,6-pentamethyl-4-piperidyl methacrylate is from 0.1 to 2 wt% of the entire amount of the above-mentioned liquid composition.

13. The liquid composition according to any one of Claims 1 to 12, wherein the content of the above-mentioned fluoroiodoalkane is from 2 to 15 wt% of the entire amount of the above-mentioned liquid composition.

14. A method of making a flammable liquid nonflammable, **characterized by** mixing a fluoroiodoalkane represented by the following formula (1): 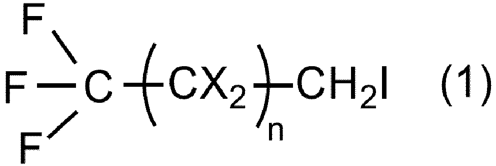 (in the formula, X each independently represents H or F, and n represents an integer of from 0 to 5) to the flammable liquid to change the above-mentioned flammable liquid to a nonflammable liquid.

15. A washing method **characterized by** bringing the liquid composition as defined in any one of Claims 1 to 13 into contact with an article to be washed to which an oily component is attached, to wash off the oily component from the above-mentioned article to be washed.

16. A disinfectant consisting of the liquid composition as defined in any one of Claims 1 to 13.
